# EUROPÄISCHE PATENTSCHRIFT

(11) **EP 1 523 311 B1**
(45) Veröffentlichungstag und Bekanntmachung des Hinweises auf die Patenterteilung: **29.03.2006**
(21) Anmeldenummer: 03765093.4
(22) Anmeldetag: 22.07.2003
(51) Int. Cl.: A61K 31/19, A61K 35/20, A61P 1/00, A61P 9/00, A61P 43/00

(54) **CALCIUM-LACTAT UND MOLKENPERMEAT ZUR SENKUNG DES TRIGLYCERIDSPIEGELS**
CALCIUM LACTATE AND WHEY PERMEATE FOR LOWERING THE TRIGLYCERIDE LEVEL
LACTATE DE CALCIUM ET PERMEAT DE LACTOSERUM DESTINES A ABAISSER LE NIVEAU DE TRIGLYCERIDE

(30) Priorität: 22.07.2002 DE 10233229
(43) Veröffentlichungstag der Anmeldung: 20.04.2005
(73) Patentinhaber: S.K. Enterprise GmbH, 20251 Hamburg (DE)
(72) Erfinder: KRAUSKOPF, Jobst, 29576 BARUM / KRS. UELZEN (DE); SOWADA, Christian, 22848 Norderstedt (DE)
(74) Vertreter: HOFFMANN EITLE
(86) Internationale Anmeldenummer: PCT/EP2003/008004
(87) Internationale Veröffentlichungsnummer: WO 2004/009070

(56) Entgegenhaltungen:
- WO-A-03/011263
- WO-A-03/011309
- DATABASE WPI Week 29 Derwent Publications Ltd., London, GB; AN 1997-311237 XP002257480 "Chromium, copper and manganese lactates and their productions and applications" & CN 1 104 625 A (ZHIANG JINGCHUN), 5. Juli 1995 (1995-07-05)

## Beschreibung

### Gebiet der Erfindung

Die vorliegende Erfindung betrifft eine pharmazeutische Zusammensetzung umfassend Ca-Lactat und Molkenpermeat zur Behandlung und/oder Prophylaxe von Hypertriglyceridämie.

### Hintergrund der Erfindung

Die koronare Herzkrankheit ist die Haupttodesursache in den industrialisierten Ländern. So ist sie für jährlich mehr als 500.000 Todesfälle in den Vereinigten Staaten verantwortlich. Es wird geschätzt, dass koronare Herzkrankheiten die Vereinigten Staaten direkt und indirekt jährlich mehr als 100 Milliarden Dollar kosten. Die primäre Ursache von koronaren Herzkrankheiten ist die Arteriosklerose, umgangssprachlich auch als Arterienverkalkung bezeichnet, eine Erkrankung, welche durch die Ablagerung von Lipiden (Cholesterine und Triglyceride) an der arteriellen Gefäßwand gekennzeichnet ist, was zu einer Einengung der Arterienöffnung und schließlich zu einer Verhärtung der Arterien führt.

Arteriosklerose, so wie sie sich in ihrer klinischen Hauptkomplikation, der koronaren Herzkrankheit oder der ischämischen Herzkrankheit manifestiert, ist weiterhin eine Haupttodesursache in industrialisierten Ländern. Es ist allgemein anerkannt, dass Arteriosklerose mit einer lokalen Verletzung am arteriellen Endothel, gefolgt von der Penetration von zirkulierenden Monocyten in das Innere der Arterienwand beginnen kann, wo sie mit aus Lipoprotein stammenden Lipiden beladen werden. Zu etwa dem gleichen Zeitpunkt scheint eine Migration von arteriellen, glatten Muskelzellen aus der Mittelschicht zur Innenschicht und deren Wachstum dort, gemeinsam mit der Ablagerung von Lipid und der Akkumulierung von Schaumzellen in der Läsion stattzufinden. Mit der Entwicklung der arteriosklerotischen Plaques verschließt diese zunehmend das betroffene Blutgefäß und kann gelegentlich zu Ischämie, Thrombose oder Infarkt führen. Es ist daher wünschenswert, Verfahren zur Verhinderung des Fortschreitens von Arteriosklerose bei Patienten, bei welchen ein Bedarf besteht, bereitzustellen.

Serumlipoproteine sind die Träger für Lipide im Kreislauf. Sie werden nach ihrer Dichte klassifiziert: Chylomicrone, Lipoproteine sehr niedriger Dichte (very low density lipoproteins,- VLDL), Lipoproteine geringer Dichte (low density lipoproteins, LDL), Lipoproteine mittlerer Dichte (intermediate density lipoproteins, IDL) und Lipoproteine hoher Dichte (high density lipoproteins, HDL). Etwa 50% bis 70% des Cholesterins, welches im Blut zirkuliert, wird in Form von LDL befördert. Im Gegensatz dazu werden etwa 25% des Gesamtcholesterins in HDL festgestellt, wogegen VLDL den Großteil der Plasmatriglyceride und nur etwa 10% bis 15% des Gesamtcholesterins trägt.

Chylomicrone werden in der Darmwand aus Produkten der Lipidverdauung zusammengestellt und anschließend über das thoracicolymphatische System in das periphere Kreislaufsystem transportiert. Im Kreislauf werden sie durch Lipoproteinlipase (LPL) in freie Fettsäuren und Triglyceride (TG) aufgebrochen, welche primär von Muskeln zur Energiegewinnung oder zur Lagerung im Fettgewebe verwendet werden. Die anderen Serumlipoproteine sind beim Transport von endogen synthetisiertem Lipid beteiligt. Der endogene Lipidtransport beginnt, wenn die Leber Triglyceride und Cholesterin in das Plasma als VLDL absondert. Die Triglyceride von VLDL werden in den Kapillaren durch LPL zu IDL und schließlich zu LDL aufgespalten. Einige dieser Teilchen werden von der Leber durch Rezeptor-übermittelte Endocytose schnell entfernt. Der Rest zirkuliert überwiegend als LDL.

Wenn Zellen absterben und sich Zellmembrane umwandeln, wird Cholesterin kontinuierlich in das Plasma freigesetzt und an HDL gebunden. HDL fördert die Entfernung von Cholesterin aus peripheren Zellen und fördert dessen Rücktransport zur Leber.

Von Hypertriglyceridämie (Hyperlipämie) spricht man bei einem erhöhten (> 160 mg/100m1) Triglyceridgehalt im Blutserum. Dieser Zustand kann eine Rolle bei der Artherogenese und der Entwicklung von koronarer Herzkrankheit spielen (Vega und Grundy, Adv. Exp. Med. 243, 311 (1989)). Zusätzlich ist eine schwere Hypertriglyceridämie (>1.000 mg/dl) mit Chylomicronämie verbunden und sie ruft akute Pankreatitis hervor (siehe K. Soergel, Acute Pancreatitis, in Gastronintestinal Disease 91, 3. Ausgabe (Sleisenger, M.H. und Fordtran, J.S., Grsg.), W.B. Saunders Company, Philadelphia, Pa., 1983, S. 1462-1485; und Brown, M.S. und Goldstein, J.L., Drugs used in the Treatment of Hyperlipoproteinemias, in Goodman and Gillman's, The Pharmacological Basis of Therapeutics 34, 7. Ausgabe, (Macmillan Publishing Co., New York, 1985, S. 827-845). Ernsthafte Erhöhungen der Chylomicrone rufen direkt Pankreatitis hervor und diese können durch Triglyceridverringerung verhindert werden (U.S. Department of Health and Human Services, NIH-Publication Nr. 89-2925, S. 74-77, Jänner 1989, "Report of the Expert Panel on Detection, Evaluation and Treatment of High Blood Cholesterol in Adults"). Es hat sich außerdem gezeigt, dass das Risiko eines Schlaganfalls bei Patienten mit koronaren Herzkrankheiten durch Verringerung des Triglyceridgehalts im Plasma vermindert werden kann (D. Tanne, et al.; Circulation 2001, 104, 2892-2897). Weiterhin ist bei Typ-2-Diabetikern die Lebenserwartung gegenüber Nichtdiabetikern um ein Drittel verkürzt. Dies steht vermutlich in direktem Zusammenhang mit einem erhöhten Triglyceridspiegel (Diabety Care; 2001- 24, 1335-1341). Es ist daher wünschenswert, ein Verfahren zur Verringerung von Plasmatriglyceriden bei Patienten mit Hypertriglyceridämie bereitzustellen.

Die Aufgabe der vorliegenden Erfindung liegt nun darin, eine pharmazeutische Zusammensetzung bereitzustellen, die zur Behandlung und/oder Prophylaxe von Hypertriglyceridämie und/oder Symptomen davon in der Lage ist.

### Beschreibung der Erfindung

Es wurde überraschend gefunden, dass die Verabreichung von Calcium-Lactat, insbesondere Calcium-L-Lactat, in Kombination mit Molkenpermeat zu einer signifikanten Verminderung der Triglycerid-Konzentration im Serum führt. Somit kann die erfindungsgemäße pharmazeutische Zusammensetzung umfassend Calcium-Lactat, insbesondere Calcium-L-Lactat, in Kombination mit Molkenpermeat verwendet werden zur Prophylaxe oder Behandlung von Hypertriglyceridämie und/oder von mit Hypertriglyceridämie einhergehenden Krankheiten bzw. Erkrankungen, insbesondere von Säugern, bevorzugt von Menschen. Bevorzugt ist in dieser Erfindung die Verwendung von Molkenpermeat, das aus Süßmolke gewonnen wurde. Beispiele von mit Hypertriglyceridämie einhergehenden Krankheiten oder Erkrankungen sind Arteriosklerose, Koronarinsuffizienz, arterielle Verschlusskrankheiten, Mykokardinfarkt, Xanthomen, Bauchbeschwerden, Milz-Leber-Vergrößerung, Pankreatis, Netzhaut-Lipämie, und/oder Schlaganfall. Auch die begleitende Gabe an Diabetes-2-Patienten ist durch die erfindungsgemäße Zusammensetzung von Vorteil.

Calcium ist ein wichtiger Nährstoff für normales Wachstum und Entwicklung. Es hilft bei der Regulierung der Zellfunktion und ist ein unverzichtbarer Strukturbaustein des Knochens. Da der Körper Calcium nicht selbst herstellen kann, muss er es über die Ernährung aufnehmen. Etwa 25 bis 35 % des durch die Nahrung aufgenommenen Calciums werden im Darm, überwiegend im Zwölffingerdarm und Leerdarm, absorbiert, die restlichen 65 bis 75 % des zugeführten Calciums werden nicht genutzt und somit ausgeschieden. Die absorbierte Menge führt hingegen zur einer Verbesserung des Knochengerüsts und somit Vorbeugung von Osteoporose. Da es sich bei Calcium außerdem um ein essentielles Element für den menschlichen Körper handelt sind keine Nebenwirkungen zu erwarten.

Lactat ist das Salz der Milchsäure (α-Hydroxypropionsäure) und existiert in der L-(+)-Form (rechtsdrehend), der D-(-)-Form und der DL-Form. Calcium-Lactat, insbesondere Calcium-L-Lactat, zeichnet sich durch eine gute Löslichkeit aus. Dadurch kann das Calcium besser absorbiert werden, was die positive Auswirkung auf das Knochengerüst erhöht. Dies verringert zwar den Anteil der im Darm absolut zur Verfügung stehenden Calciummenge, dem kann jedoch einfach durch Erhöhung der aufgenommenen Menge entgegengewirkt werden. Außerdem ist die Calciummenge im menschlichen Körper ein kontinuierlicher Ausgleich zwischen Absorption und Ausscheidung. Das vermehrt aufgenommene Calcium wird somit zum größten Teil wieder ausgeschieden.

Molke fällt bei der Käseherstellung aus Milch, wie z.B. Kuhmilch, Ziegenmilch, Schafsmilch, Büffelmilch und Kamelmilch, an, nachdem das in der Milch vorhandene Casein ausgefällt wurde. Nach Art der Gewinnung unterscheidet man Süßmolke, die als Milchserum nach enzymatischer Ausfällung von Casein durch Labferment entsteht, und Sauermolke, die nach Abtrennung des Caseins durch Säurefällung gewonnen wird. Die pH-Wert-Grenze zwischen Süß- und Sauermolke ist nicht ganz scharf umrissen und liegt allgemein über bzw. unter einem Bereich von 5,6 bis 5,9.

Molke enthält alle wasserlöslichen Komponenten der Milch, insofern diese nicht durch Lab oder Säure ausgefällt werden. Süßmolke enthält ca. 4,9% Lactose, 0,8% Protein, 0,5% Mineralstoffe, 0,2% Milchfett und 0,2% Milchsäure.

Aus Molke bzw. Süßmolke kann mit Hilfe der Ultrafiltration (Membranverfahren, mittlere Porengröße: 25 bis 100 kDalton) das Molkeneiweiß abgetrennt werden. Diese "enteiweißte" Molke besteht zu etwa 95% aus Wasser und kann durch Sprühtrocknen zu einem Pulver weiterverarbeitet werden kann. Dieses Pulver wird hier als Molkenpermeat bezeichnet. In der vorliegenden Erfindung ist die Verwendung von Molkenpermeat, das aus Süßmolke gewonnen wurde, bevorzugt. Seine durchschnittlichen Bestandteile sind 84.9% Lactose, 4.5% Protein, 0.1% Fett und 7.5% Mineralstoffe. Beim Rest handelt es sich um nichtabgetrenntes Wasser. Bei diesen Mengenangaben handelt es sich um Durchschnittswerte, die je nach Herstellungsweise um 5-10% (relativ) variieren können. Molkenpermeat kann als solches, z.B. in Pulverform, oder nach (Teil-)Hydrolyse der Lactose als Sirup oder Pulver verwendet werden.

In einer weiteren erfindungsgemäßen Ausführungsform hat sich eine Mikroverkapselung der pharmazeutischen Zusammensetzung umfassend Calcium-Lactat und Molkenpermeat als besonders vorteilhaft gezeigt. Die Mikroverkapselung kann z.B. wie in den Patent-Offenlegungsschriften DE 198 54 749 A1 und DE 100 08 880 A1 und dem Gebrauchsmuster DE 296 23 285 U1 beschrieben, erfolgen. Dabei wird die Verbindung zum Beispiel in einer Hülle aus einem Polysaccharid, wie z.B. Alginat, fest eingeschlossen. Damit der möglicherweise unverdauliche Hüllstoff eine Freisetzung der Verbindung nicht verhindert und dadurch eine ernährungsphysiologische Nutzung durch den Organismus unmöglich macht, kann eine verdauliche Komponente, wie z.B. Stärke der Umhüllung beigefügt werden. Durch geschickte Wahl und/oder Kombination der löslichen und unlöslichen Umhüllungskomponenten kann so die Abgabe der mikroverkapselten pharmazeutischen Zusammensetzung in verschiedenen Bereichen des Verdauungstrakts gezielt gesteuert werden. Eine abgestufte Freisetzung im Darm, z.B. eine Freisetzung von 50 bis 80 Gew.-%, bevorzugt von 60 bis 70 Gew.-%, insbesondere von 62,5 Gew.-% im Dünndarm, und eine Freisetzung von 20 bis 50 Gew.-%, bevorzugt von 30 bis 40 Gew.-%, insbesondere von 62,5 Gew.-% im Dickdarm ist eine mögliche Art der gezielten Freisetzung. Ein weiterer vorteilhafter Effekt kann durch eine verlängerte Haltbarkeit durch Schutz der verkapselten Verbindung z.B. vor Umwelteinflüssen erzielt werden.

Die pharmazeutische Zusammensetzung wird bevorzugt so dosiert, dass die Dosis pro Gabe 1,0 g bis 15.0 g, bevorzugt 5.0 g bis 10.0 g, am bevorzugtesten 6.0 g bis 9.0 g beträgt. Die Verabreichung geschieht vorteilhafterweise in Dosierungen von einmal bis sechsmal täglich, wobei eine ein- bis viermal tägliche Dosis bevorzugt ist. Die orale Gabe hat sich als vorteilhaft erwiesen, insbesondere in den oben genannten Mengen in 100 ml bis 300 ml, vorzugsweise in 125 ml oder 200 ml einer Getränkezubereitung. Das tatsächliche Dosisintervall und die Dosismenge hängt jedoch ab von Faktoren wie z.B. Zustand, Alter, Gewicht, und/oder Geschlecht des Behandelten, die von Individuum zu Individuum variieren können. Insbesondere bei Schwangeren und Stillenden ist eine erhöhte Einnahmemenge der pharmazeutischen Zusammensetzung vorteilhaft. Ein zusätzlicher vorteilhafter Einfluss kann sich z.B. auch durch Einnahme des Präparats kurz vor oder während der Mahlzeiten ergeben. Bei der Verabreichung des Präparats an Säuger im allgemeinen erfolgt die Dosierung ebenfalls in Abhängigkeit von Tierart und Gewicht.

Erfindungsgemäß kann die pharmazeutische Zusammensetzung umfassend Calcium-Lactat und Molkenpermeat verwendet werden zur Herstellung von Lebensmitteln, wie z.B. diätetischen Lebensmitteln und/oder Nahrungsergänzungsmitteln, wodurch diese die Eigenschaft erhalten, dass sie bei oraler Verabreichung eine Absenkung des Triglyceridgehalts bewirken können. Beispiele für derartige Lebensmittel sind mit der pharmazeutischen Zusammensetzung angereicherte Milchprodukte oder Fruchtsäfte.

Die Erfindung wird im Folgenden durch Experimente näher erläutert, welche nicht den Bereich der Erfindung weiter einschränken sollen.

### Experimente

Sowohl pharmakologische als auch ernährungsinduzierte Interventionsstudien bei Patienten mit erhöhten Triglyceridspiegel sind vor allem zeitaufwendig, um Veränderungen des Stoffwechsels nachzuweisen und erfordern eine hohe Disziplin und Motivation der an den Studien teilnehmenden Probanden bzw. Patienten. Partiell können diese Nachteile durch Tierversuche vermindert und/oder ausgeschlossen werden, vorausgesetzt, dass äquivalente Tiermodelle zur Verfügung stehen. Im Gegensatz zu den meisten in der Literatur beschriebenen Modelltieren, die monogenetisch-dominant vererbbare Störungen aufweisen (Zucker-Ratte, Kolesky-Ratte, hHTG-Ratte), stellt die an der Universität Greifswald (Abteilung für Versuchstierkunde) seit 1995 untersuchte, charakterisierte und ingezogene WOK.W-Ratte ein Tiermodell dar, das polygentisch die Störung vererbt und u.a. einen erhöhten Triglyceridspiegel entwickelt, wobei männliche Tiere diese Symptome stärker exprimieren. Die spezifischen Eigenschaften der WOK.W-Ratten sind z.B. beschrieben in:
P.Kovacs et al., *Ann.N.Y.Acad.Sci.,* **1997**, *827,* 94-99;
P.Kovacs et al., *Biochem.Biophsy.Res.Commun.,* **2000**, 660-665;
J.van den Brandt et al., *Int.J.Obesity,* **2000**, *24*, 1618-1622;
J.van den Brandt et al., *Metabolism 49*, **2000**, 1140-1144.

Dieses Modelltier ist dafür geeignet, eine exogene Modulation als Interventionsstudie bei dieser Erkrankung zu untersuchen, zumal vorläufige Untersuchungen belegen, dass beispielsweise durch fettreiche Ernährung die Erkrankungsinzidenz verändert werden kann (Tabelle 1).

**Tabelle 1: Vergleich ausgewählter Parameter von Patienten und WOK.W-Ratten**

| **Parameter** | ***WOK.W-Ratte*** | **MENSCH** |
|---|---|---|
| Fettsucht | +++¹ | +++ |
| Hypertriglyceridämie | +++ | +++ |
| Hypercholesterinämie | + | ++² |
| Dyslipoproteinämie | ++ | +++ |
| Vermindertes HDL-Cholesterin | +³ | ++ |
| Hyperleptinämie | +++ | +++ |
| Glukoseintoleranz | ++ | +++ |
| Insulinresistenz | ++ | +++ |
| Hyperinsulinämie | ++ | ++ |
| Hypertonie | + | +++ |

| | | |
|---|---|---|
| 1: +++ = sehr stark ausgeprägt, 2:++ = stark ausgeprägt, 3: + = vorhanden | | |

### Versuchsprogramm

31 männliche WOK.W-Ratten (aus 8 Würfen) wurden in einem Alter von 4 Wochen in 4 Gruppen so aufgeteilt, dass jeweils 1 Geschwistertier in einer Gruppe vertreten war. Gruppe 1 (n=8 Tiere) wurde *ad libitum* ernährt und getränkt und diente als unbehandelte Kontrollgruppe. Gruppe 2 (n=7 Tiere) erhielt mit Molkenpermeat versetztes Trinkwasser (25 g Süßmolkenpermeat/1000 ml Trinkwasser) eingeflößt, wobei als Grundlage für die Kalkulation ein täglicher Trinkwasserbedarf von durchschnittlich 20 ml angenommen wurde. Die Gruppe 3 (n=8 Tiere) erhielt mit Calcium-L-Lactat versetztes Trinkwasser (5,6 g Calcium-L-Lactat/1000 ml Trinkwasser), und die Gruppe 4 (n=8 Tiere) erhielt Molkenpermeat und Calcium-L-Lactat in identischen Konzentrationen. Der Behandlungsversuch begann in einem Alter von 4 Wochen, d.h. vor der Ausprägung der wichtigsten Symptome des metabolischen Syndroms, und dauerte 12 Wochen, bis zu einem Alter von 16 Wochen.

### Parameter

Die Körpermasse, die Körperlänge (zur Berechnung des Körpermasseindex, BMI), die Plasmaglukose, und die Serumtriglyceride wurden alle 2 Wochen erfasst. Zum Versuchsende wurde Serum gewonnen und eingefroren und außerdem das Pankreas präpariert und in flüssigem Stickstoff gefroren bzw. in Bouin'scher Lösung fixiert, um nachfolgend eventuell morphologische Untersuchungen zu ermöglichen. Außerdem wurde das pararenale Fettgewebe präpariert und gewogen.

Die Plasmaglukose wurde enzymatisch mittels Glukoseanalysator gemessen. Die Fettstoffwechselparameter wurden enzymatisch am klinisch-chemischen Analyseautomaten mit Kits (Boehringer Mannheim: Triglyceride mit TG GPO-PAP) entsprechend der mitgelieferten Arbeitsvorschriften bestimmt.

Die pathologischen Grenzwerte liegen für Plasmaglukose >8,4 mmol/1, und für die Serumtriglyceride >2,3 mmol/1.

Die Ergebnisse werden als Mittelwert ± SEM dargestellt und die statistische Bedeutsamkeit wurde mittels Student's t-test errechnet.

### Ergebnisse

Durch die gewählte Behandlungsform wird die Köpermasse (als Ausdruck der Fettsucht) nicht beeinflusst, ebenso wie der BMI und die Plasmaglukosekonzentrationen sich nicht verändern.

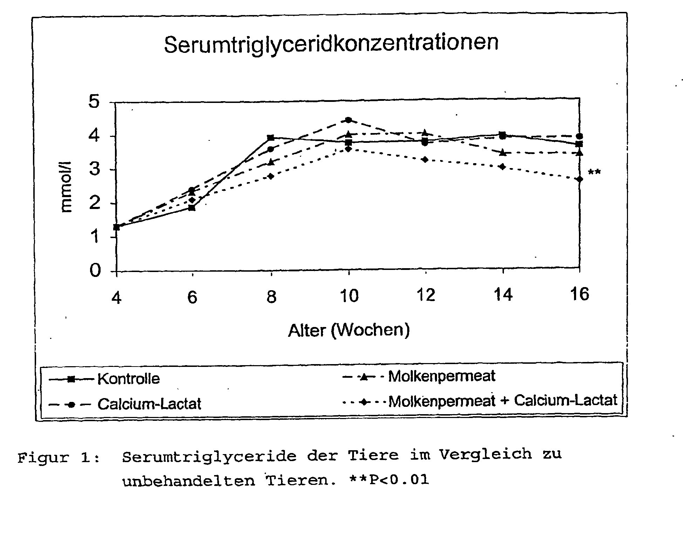

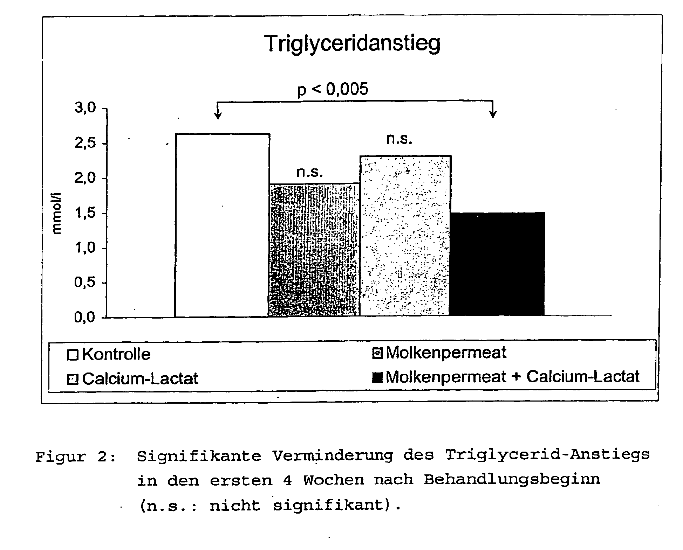

Die pathogenetisch bedeutsamen Serumtriglyceride steigen in allen Versuchstiergruppen an (Figur 1) und überschreiten den pathologischen Grenzwert im Alter von 6-8 Wochen. Der Anstieg der TG's (Figur 2) und die absoluten TG-Konzentrationen sind signifikant geringer in der Versuchsgruppe 4, obwohl in allen Gruppen keine Normwerte erreicht werden konnten. Die singuläre Behandlung der Tiere modulierte die TG-Konzentrationen zu keiner Zeit (Figur 1).

Die Ergebnisse der untersuchten Parameter wurden in Tabelle 2, zum Versuchsende erhoben, zusammengefasst.

**Tabelle 2: Zusammenfassung der Ergebnisse des Fütterungsversuches (Männliche WOK.W-Ratten, 16 Wochen alt) nach Beendigung des Nahrungsergänzung; ^{a}P<0,01, ^{b}p<0,001**

| Parameter | Dimension | Kontrolle | Molkenpermeat | calcium-Lactat | Molkenpermeat + Calcium-Lactat |
|---|---|---|---|---|---|
| Anzahl | N | 8 | 7 | 8 | 8 |
| Körpermasse | G | 394,0 ± 10,2 | 406,3 ± 9.2 | 394,3 ± 10,6 | 385,0 ± 23,5 |
| BMI | g/cm² | 0,78 ± 0,01 | 0,76 ± 4,01 | 0,77 ± 0,01 | 0,75 ± 0,02 |
| Fettgewebe | G | 11,50 ± 0,60 | 11,45 0,57 | 10,01 ± 0,71 | 10,25 ± 1,06 |
| Blutzucker | mmol/l | 5,61 ± 0,11 | 6,05 ± 0,26 | 6,10 ± 0,21 | 5,64 ± 0,28 |
| Triglyceride | mmol/l | 3,65 ± 0,18 | 3,39 ± 0,51 | 3,88 ± 0,22 | 2,60 ± 0,28^{a} |

### Schlussfolgerungen

Die Studie belegt, dass die kombinierte Behandlung Fettstoffwechselparameter bei Ratten mit erhöhtem Triglyceridspiegel signifikant beeinflussen kann. Dies eröffnet die Möglichkeit der Prophylaxe oder Behandlung von Hypertriglyceridämie und/oder Symptomen davon durch kombinierte Gabe von Calcium-Lactat und Molkenpermeat an Säuger, insbesondere an Menschen.

## Patentansprüche

1. Verwendung von Calcium-Lactat, insbesondere Calcium-L-Lactat, und Molkenpermeat, zur Herstellung einer pharmazeutischen Zusammensetzung zur Prophylaxe oder Behandlung eines erhöhten Triglyceridspiegels bei einem Säuger, insbesondere beim Menschen.

2. Verwendung von Calcium-Lactat, insbesondere Calcium-L-Lactat, und Molkenpermeat, zur Herstellung einer pharmazeutischen Zusammensetzung zur Prophylaxe oder Behandlung von Arteriosklerose, Koronarinsuffizienz, arterielle Verschlusskrankheiten, Mykokardinfarkt, Xanthomen, Bauchbeschwerden, Milz-Leber-Vergrößerung, Pankreatitis, Netzhaut-Lipämie und/oder Schlaganfall, und/oder damit verbundener Krankheiten oder Erkrankungen beim säuger, insbesondere beim Menschen.

3. Verwendung gemäß Anspruch 1 oder 2, worin die pharmazeutische Zusammensetzung zur gleichzeitigen Verbesserung des Knochengerüsts und/oder zur gleichzeitigen Prophylaxe und/oder Behandlung von Osteoporose dient.

4. Verwendung gemäß einem der Ansprüche 1 bis 3, worin das Molkenpermeat aus Süßmolke gewonnen wird.

5. Verwendung gemäß einem der Ansprüche 1 bis 4, worin das Molkenpermeat hydrolysiert ist.

6. Verwendung gemäß einem der Ansprüche 1 bis 5, worin das Molkenpermeat teilhydrolysiert ist.

7. Verwendung gemäß einem der Ansprüche 1 bis 6, worin die Zusammensetzung mikroverkapselt ist.

8. Verwendung gemäß einem der Ansprüche 1 bis 7, worin die Zusammensetzung der oralen Gabe dient.

9. Verwendung gemäß einem der Ansprüche 1 bis 8, worin die Dosis pro Gabe 1,0 g bis 15,0 g, bevorzugt 5,0 g bis 10,0 g, am bevorzugtesten 6,0 g bis 8,0 g beträgt.

10. Verwendung gemäß einem der Ansprüche 1 bis 9, wobei das Calcium-Lactat durch Fermentation gewonnen wird.

11. Verwendung gemäß einem der Ansprüche 1 bis 10, wobei die Zusammensetzung ferner Zusatzstoffe enthält, die zur Herstellung von Lebensmitteln, insbesondere diätetischen Lebensmitteln, und/oder Nahrungsergänzungsmitteln geeignet sind.

12. Verwendung gemäß einem der Ansprüche 1 bis 11, wobei die Zusammensetzung ferner pharmazeutisch annehmbare Zusatzstoffe und/oder Trägermaterialien umfasst.

13. Verwendung gemäß einem der Ansprüche 1 bis 12, wobei die Zusammensetzung in fester Form als Lutschtablette, Pulver oder Granulat, oder in flüssiger Form als Sirup oder Saft vorliegt.

14. Pharmazeutische Zusammensetzung umfassend Calcium-Lactat, insbesondere Calcium-L-Lactat, und Molkenpermeat.

## Claims

1. Use of calcium lactate, in particular calcium L-lactate, and whey permeate, for producing a pharmaceutical composition for the prophylaxis or treatment of an elevated triglyceride level in a mammal, in particular in a human being.

2. Use of calcium lactate, in particular calcium L-lactate, and whey permeate, for producing a pharmaceutical composition for the prophylaxis or treatment of arteriosclerosis, coronary insufficiency, arterial occlusive vascular diseases, myocardial infarction, xanthoma, abdominal complaints, spleen-liver enlargement, pancreatitis, retinal lipaemia and/or stroke, and/or diseases or conditions associated therewith in a mammal, in particular in a human being.

3. Use according to claim 1 or 2, wherein the pharmaceutical composition serves for simultaneous improvement of the skeleton and/or for the simultaneous prophylaxis and/or treatment of osteoporosis.

4. Use according to one of claims 1 to 3, wherein the whey permeate is recovered from sweet whey.

5. Use according to one of claims 1 to 4, wherein the whey permeate is hydrolysed.

6. Use according to one of claims 1 to 5, wherein the whey permeate is partially hydrolysed.

7. Use according to one of claims 1 to 6, wherein the composition is microencapsulated.

8. Use according to one of claims 1 to 7, wherein the composition serves for oral administration.

9. Use according to one of claims 1 to 8, wherein the dose per administration is 1.0 g to 15.0 g, preferably 5.0 g to 10.0 g, most preferably 6.0 g to 8.0 g.

10. Use according to one of claims 1 to 9, wherein the calcium lactate is recovered by fermentation.

11. Use according to one of claims 1 to 10, wherein the composition also contains additives which are suitable for the production of foodstuffs, in particular dietetic foodstuffs, and/or nutritional supplements.

12. Use according to one of claims 1 to 11, wherein the composition also comprises pharmaceutically acceptable additives and/or excipient materials.

13. Use according to one of claims 1 to 12, wherein the composition exists in solid form as a sucking tablet, powder or granules, or in liquid form as syrup or juice.

14. Pharmaceutical composition comprising calcium lactate, in particular calcium L-lactate, and whey permeate.

## Revendications

1. Utilisation de lactate de calcium, en particulier de lactate L de calcium et de perméat de lactosérum, pour la préparation d'une composition pharmaceutique pour la prophylaxie ou le traitement d'une augmentation du niveau de triglycéride, chez un nourrisson allaité, en particulier chez l'humain.

2. Utilisation de lactate de calcium, en particulier de lactate L de calcium et de perméat de lactosérum, pour la préparation d'une composition pharmaceutique de prophylaxie ou de traitement de l'artériosclérose, de l'insuffisance coronarienne, des maladies de l'obstruction artérielle, de l'infarctus du myocarde, du xanthome, des maux abdominaux, du grossissement de la rate et du foie, de la pancréatique, de la lipémie de la rétine, et/ou d'une apoplexie cérébrale et/ou des maladies ou des infections leur étant liées chez un nourrisson allaité, en particulier chez l'humain.

3. Utilisation selon la revendication 1 ou 2, la composition pharmaceutique servant à l'amélioration simultanée de la charpente osseuse, et/ou à la prophylaxie et/ou au traitement simultané de l'ostéoporose.

4. Utilisation selon l'une des revendications 1 à 3, dans lequel le perméat de lactosérum étant obtenu à partir de petit-lait doux.

5. Utilisation selon l'une des revendications 1 à 4, dans lequel le perméat de lactosérum est hydrolysé.

6. Utilisation selon l'une des revendications 1 à 5, dans lequel le perméat de lactosérum est partiellement hydrolysé.

7. Utilisation selon l'une des revendications 1 à 6, dans lequel la composition est micro-encapsulée.

8. Utilisation selon l'une des revendications 1 à 7, la composition servant à l'administration orale.

9. Utilisation selon l'une des revendications 1 à 8, dans lequel la dose par administration est de 1,0 g à 15,0 g, de préférence de 5,0 g à 10,0 g, de manière la mieux préférée de 6,0 g à 8,0 g.

10. Utilisation selon l'une des revendications 1 à 9, le lactate de calcium étant obtenu par fermentation.

11. Utilisation selon l'une des revendications 1 à 10, la composition contenant en outre des additifs convenant pour la préparation de produits alimentaires, en particulier de produits alimentaires diététiques, et/ou de produits de compléments d'alimentation.

12. Utilisation selon l'une des revendications 1 à 11, la composition comprenant en outre des additifs et/ou des matériaux supports, acceptables pharmaceutiquement.

13. Utilisation selon l'une des revendications 1 à 12, la composition se présentant sous forme solide, sous forme de comprimés à sucer, de poudre ou de granulé, ou bien sous forme liquide, sous forme de sirop ou de jus.

14. Composition pharmaceutique comprenant du lactate de calcium, en particulier du lactate L de calcium et du perméat de lactosérum.
